(19) Europäisches Patentamt
European Patent Office
Office européen des brevets

(11) Publication number: 0 107 350 B1

(12) # EUROPEAN PATENT SPECIFICATION

(45) Date of publication of patent specification: **30.04.86**

(51) Int. Cl.$^4$: **C 07 D 295/12,** C 07 D 207/06, C 07 D 211/14, A 61 K 31/395

(21) Application number: **83305644.3**

(22) Date of filing: **22.09.83**

(54) **Sulfon amides.**

(30) Priority: **28.09.82 GB 8227841**

(43) Date of publication of application: **02.05.84 Bulletin 84/18**

(45) Publication of the grant of the patent: **30.04.86 Bulletin 86/18**

(84) Designated Contracting States: **BE CH DE FR GB IT LI NL SE**

(58) References cited:
**EP-A-0 021 592**
**EP-A-0 064 445**
**EP-A-0 076 072**

(73) Proprietor: **Beecham Wuelfing GmbH & Co KG**
**PO Box 25 Stresemannallee 6**
**D-404 Neuss (DE)**

(72) Inventor: **Jozic, Ljerka**
**An der Bismarck-Schule. 4B**
**D-3000 Hannover 1 (DE)**

(74) Representative: **Stott, Michael John et al**
**European Patent Attorney Beecham Pharmaceuticals Great Burgh Yew Tree Bottom Road**
**Epsom Surrey KT18 5XQ (GB)**

**EP 0 107 350 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel substituted sulphonamides having useful pharmacological properties, to pharmaceutical compositions containing them, to a process for their preparation, to intermediates for their preparation, and to the compounds for therapeutic or prophylactic use.

EP—A 21592 discloses a pharmaceutical composition which comprises a compound of the formula (A):

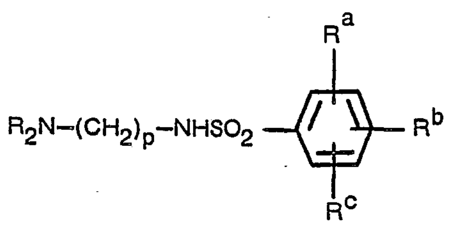

(A)

or a salt thereof wherein $NR_2$ represents a piperidyl, pyrrolidyl, morpholino or N-methyl piperazyl group, any of which may be substituted by one or two methyl groups; $R^a$ represents fluorine, chlorine, bromine, iodine, lower alkoxyl, hydroxyl, acetoxyl, nitro, cyano, amino, dimethylamino, lower alkyl, trifluoromethyl, methylthio, ethylthio, methoxycarbonyl, ethoxycarbonyl, acetamido or carboxamido; $R^b$ represents hydrogen, fluorine, chlorine, bromine, iodine, lower alkoxyl, hydroxyl, lower alkyl; $R^c$ represents hydrogen, chlorine or lower alkyl; or $R^a$ and $R^b$ when on adjacent carbon atoms may together represent a polymethylene group containing from 3 to 5 carbon atoms; and p is 2, 3 or 4; and a pharmaceutically acceptable carrier. The compounds are antiarrhythmic agents.

It has now been found that a structurally distinct class of substituted sulphonamides has useful pharmacological activity, in particular cardiovascular activity such as antiarrythmic activity.

Accordingly, the present invention provides a compound of the formula (I) or a pharmaceutically acceptable salt or N- oxide thereof, or a solvate of any of the foregoing:

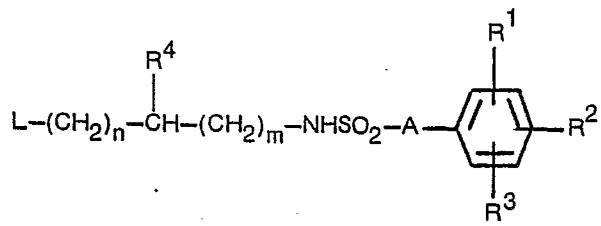

(I)

wherein:

A is a bond, $—CH_2—$ or $—CH{=}CH—$;

m is 0 or 1;

n is 0 to 3;

$R^4$ is $C_{1-4}$ alkyl or optionally derivatised hydroxy;

$R^1$ is halo, $C_{1-4}$ alkyl, hydroxy, $C_{1-4}$ alkanoyl, nitro, cyano, $CF_3$ or amino optionally substituted by one or two $C_{1-4}$ alkyl groups; and

$R^2$ and $R^3$ are each independently halo, $C_{1-4}$ alkyl, or hydroxy; and

L is 1-pyrrolidyl, 1-piperidyl, 1-morpholinyl or 4-methyl-1-piperazyl, optionally singly substituted as appropriate by a methyl group at each carbon atom in the 2-, 4- or 6-position up to a total of two methyl substituents in the radical L.

A is preferably a bond.

m may be either 0 or 1. n may be any value from 0 to 3, more suitably 1, 2 or 3, favourably 1 or 3.

Apt values for $R^4$ $C_{1-4}$ alkyl include methyl ethyl and isopropyl, preferably methyl.

When used herein, derivatised hydroxy $R^4$ are nitrato, $C_{1-4}$ alkoxy, phenyl $C_{1-4}$ alkoxy, or *in vivo* hydrolysable acyloxy.

Apt values for $R^4$ $C_{1-4}$ alkoxy groups include methoxy, ethoxy and n- and iso-propoxy, preferably methoxy.

Examples of $R^4$ *in vivo* hydrolysable acyloxy groups include $C_{1-6}$ alkanoyloxy, for example acetoxy, propionoxy, *n*- and *iso*-butyroxy, and 2,3-dimethylpropanoyloxy; benzoyloxy or benzenesulphonyloxy either being optionally substituted in the phenyl ring by one or two substituents selected from $C_{1-4}$ alkyl, $C_{1-4}$ alkoxy, trifluoromethyl, halogen or nitro; $C_{1-6}$ alkanesulphonyloxy group, such as methane-sulphonyloxy; $C_{1-6}$ alkoxycarbonyloxy, such as tert.- butoxycarbonyloxy; and carbamoyloxy optionally N-substituted by $C_{1-6}$ alkyl, such as methylcarbamoyloxy.

A group of variables $R^1$ consists of $C_{1-4}$ alkyl, $C_{1-4}$ alkanoyl, nitro, cyano and $CF_3$, in particular $C_{1-4}$ alkyl. A second group of variables $R^1$ consists of halo and hydroxy, in particular halo. Suitable examples of $R^1$ include methyl, ethyl and isopropyl. Suitable examples of $R^1$ also include fluoro, chloro, bromo and iodo.

Preferred examples of $R^1$ include methyl. Preferred examples of $R^1$ also include chloro. $R^1$ is often in the 2-position.

A group of variables $R^2$ consists of $C_{1-4}$ alkyl. A second group of variables $R^2$ consists of halo and

hydroxy, in particular halo. Suitable examples of $R^2$ include methyl, ethyl, and isopropyl. Suitable examples of $R^2$ also include fluoro, chloro, bromo and iodo.

Preferred examples of $R^2$ include methyl. Preferred examples of $R^2$ also include chloro. $R^2$ is often in the 4- position.

A group of variables $R^3$ consists of $C_{1-4}$ alkyl. A second group of variables $R^3$ consists of halo. Suitable examples of $R^3$ include methyl, ethyl and isopropyl. Suitable examples of $R^3$ also include fluoro, chloro, bromo and iodo.

Preferred examples of $R^3$ include methyl. Preferred examples of $R^3$ also include chloro. $R^3$ is often in the 5- or 6- position.

Preferably $R^1$, $R^2$ and $R^3$ are the same.

A group of values for L consists of 1-pyrrolidyl, 3-methyl-1-pyrrolidyl and 2,4-dimethyl-1-pyrrolidyl. A second group of values for L consists of 1-piperidyl, 2-methyl-1-piperidyl, 1-morpholinyl and 4-methyl-1-piperazyl.

Favoured values for L include 2,4-dimethyl-1-pyrrolidyl. Favoured values for L also include 1-piperidyl, 2-methyl-1-piperidyl, 1-morpholinyl and 4-methyl-1-piperazyl.

Especially favoured values for L include 1-piperidyl and 2-methyl-1-piperidyl. Preferred L include 2-methyl-1-piperidyl.

In a group of compounds within formula (I):

$R^1$ is halogen, hydroxy, $C_{1-4}$ alkanoyl, nitro, cyano, $C_{1-4}$ alkyl, $CF_3$ or amino optionally substituted by one or two $C_{1-4}$ alkyl groups;

$R^2$ is halogen, hydroxy or $C_{1-4}$ alkyl; and either.

$R^3$ is halogen or $C_{1-4}$ alkyl; and

L is 1-piperidyl, 1-morpholinyl, or 4-methyl-1-piperazyl, optionally substituted by one or two methyl groups in the 2-, 4- or 6- positions as appropriate; or

$R^3$ is $C_{1-4}$ alkyl; and

L is 1-pyrrolidyl optionally substituted by one or two methyl groups in the 2- or 4-positions;

$R^4$ is $C_{1-4}$ alkyl, hydroxy, $C_{1-4}$ alkoxy, $C_{1-4}$ alkanoyloxy or nitrato;

From the foregoing it will be realised that a group of compounds of the formula (I) is of formula (II):

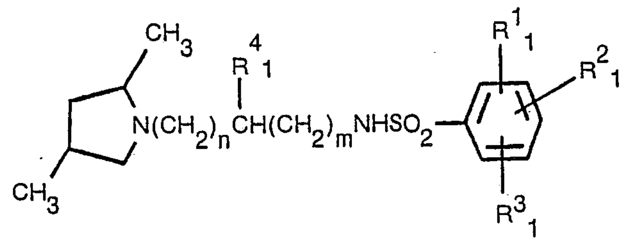

(II)

wherein:

each of $R^1_1$, $R^2_1$ and $R^3_1$ is independently halogen or $C_{1-4}$ alkyl.

$R^4_1$ is methyl or hydroxy; and the remaining variables are as defined in formula (I).

Suitable and preferred n are as so described hereinbefore. Suitable and preferred $R^1_1$, $R^2_1$ and $R^3_1$ and $R^4_1$ are as so described for relevant $R^1$, $R^2$ and $R^3$ and $R^4$ hereinbefore.

A preferred sub-group of formula (II) is of formula (III):

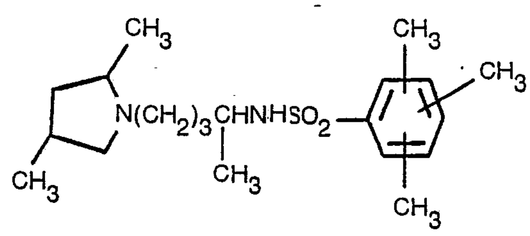

(III)

The 2,4,6-trimethylphenyl nucleus is preferred.

A second sub-group of formula (II) is of formula (IV);

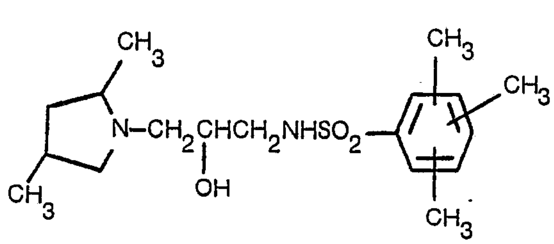

(IV)

The 2,4,6-trimethylphenyl nucleus is preferred.

The second group of compounds of formula (I) is of formula (V):

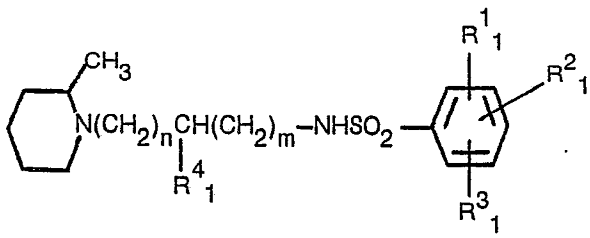

(V)

wherein the variables are as hereinbefore defined.

Suitable and preferred variables are as so described under formula (II).

A preferred sub-group of formula (V) is of formula (VI):

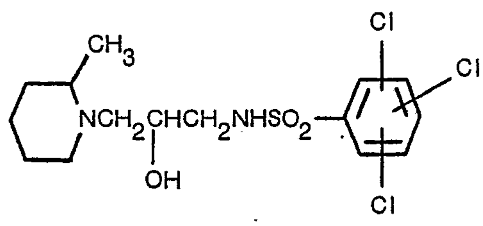

(VI)

The 2,4,5-trichlorophenyl nucleus is preferred.

A second preferred sub-group of formula (V) is of formula (VII):

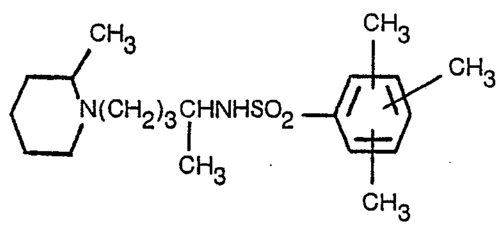

(VII)

The 2,4,6-trimethylphenyl nucleus is particularly preferred.

It will of course be realised that when L in the compounds of the invention is asymmetrically substituted by one or two methyl groups, the L group has a chiral centre. The compounds of the formula (I) also contain a chiral centre at the point of substitution by an $R^4$ group. When L is substituted by two methyl groups, these may give rise to different isomers wherein the two groups are mutually cis or trans across the ring. The compounds may thus be provided in a number of isomeric forms or in mixtures thereof. A mixture of isomers containing all the possible forms of a given compound is particularly apt, in view of its greater ease of synthesis.

The invention extends to any of the stereoisomeric forms, including enantiomers, of the compounds of the invention and to mixtures thereof, including racemates. The different stereoisomeric forms may be separated or resolved one from the other by the usual methods, or any given isomer may be obtained by stereospecific or asymmetric synthesis.

The invention extends in one aspect to pharmaceutically acceptable salts of the compounds of the formula (I). These include acid addition salts with conventional acids such as hydrochloric, hydrobromic boric, phosphoric, sulphuric and pharmaceutically acceptable organic acids such as acetic, tartaric, maleic, citric, succinic, benzoic, ascorbic, methanesulphonic, α-keto-glutaric, α-glycerophosphoric, and glucose-1-phosphoric acids. Preferably the acid addition salts is a hemisuccinate or hydrochloride, in particular the hydrochloride salt.

Pharmaceutically acceptable salts also include quaternary salts. Examples of quaternary salts include such compounds quaternised by compounds such as $R^8$—T wherein $R^8$ is $C_{1-6}$ alkyl, phenyl-$C_{1-6}$ alkyl or $C_{5-7}$ cycloalkyl, and T is a radical corresponding to an anion of an acid. Suitable examples of $R^8$ include methyl, ethyl and *n*- and *iso*-propyl; and benzyl and phenethyl. Suitable T include halide such as chloride, bromide and iodide.

The pharmaceutically acceptable salts of the compounds of the formula (I) are usually acid addition salts with conventional acids such as hydrochloric, hydrobromic, phosphoric, sulphuric, citric, tartaric, lactic and acetic acid, in particular the hydrochloride salt.

The compounds of formula (I) may also form pharmaceutically acceptable N-oxides, and the invention extends to these.

The compounds of the formula (I) and their pharmaceutically acceptable salts and N-oxides may also form solvates with pharmaceutically acceptable solvents and the invention extends to these.

It will also be realised that salts of the compounds of the formula (I) which are not pharmaceutically acceptable may be useful as intermediates in the preparation of pharmaceutically acceptable salts of compounds of the formula (I) or the compounds of the formula (I) themselves, and as such form an aspect of the present invention.

It is preferred that the compounds of the present invention are in substantially pure form, for example in greater than 95% purity, in particular greater than 98% purity. It is also preferred that the compounds of the present invention are in crystalline form.

Crystalline pharmaceutically acceptable acid addition salts and their solvates are favoured in view of their enhanced stability.

The compounds of formula (I) have cardiovascular, in particular antiarrhythmic, activity. Although some of the compounds contain a substituted propanolamine chain, it is believed that they have minimal β-blockade on the heart or bronchioles.

In order to utilise the activity of the compounds of formula (I) the invention also provides a pharmaceutical composition comprising a compound of formula (I), or a pharmaceutically acceptable salt thereof, or N-oxide thereof, or a solvate of any of the foregoing, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate. Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl *p*-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as anti-hypertensive agents and diuretics.

As is common practice, the compositions will usually be accompanied by written or printed directions for use in the medical treatment concerned.

The invention further relates to a method of treatment or prophylaxis of cardiac arrhythmia in mammals, such as humans, which comprises the administration of an effective amount of a compound of formula (I), or a pharmaceutically acceptable salt thereof, or N-oxide thereof, or a solvate of any of the foregoing as hereinbefore defined, to the sufferer.

An amount effective to treat the disorders hereinbefore described depends on the relative efficacies of the compounds of the formula (I), the nature and severity of the disorders being treated and the weight of the mammal. However, a unit dose will normally contain 1 to 100 mg for example 2 to 50 mg, of the compound of the invention. Unit doses will normally be administered once or more than once a day, for

example 2, 3, 4, 5 or 6 times a day, more usually 2 to 4 times a day, such that the total daily dose is normally in the range, for a 70 kg adult of 0.1 to 250 mg, more usually 50 to 200 mg, for example 10 to 75 mg, that is in the range of approximately 0.002 to 35 mg/kg/day, more usually 1 to 30 mg/kg/day, for example 0.15 to 1 mg/kg/day.

The invention also provides a compound of formula (I), or a pharmaceutically acceptable salt thereof, or N-oxide thereof, or a solvate of any of the foregoing as hereinbefore defined, for therapeutic or prophylactic use, in particular for the treatment or prophylaxis of cardiac arrhythmia.

The present invention also provides a process for the preparation of a compound of formula (I) or a pharmaceutically acceptable salt or N-oxide thereof, or a solvate of any of the foregoing, which process comprises the reaction of the compounds of formulae (VIII) and (IX):

L−V (VIII)

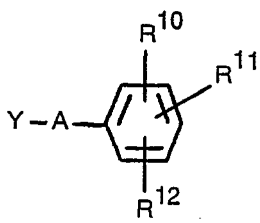

(IX)

wherein

L is as hereinbefore defined;

$R^{10}$, $R^{11}$ and $R^{12}$ are $R^1$, $R^2$ and $R^3$ respectively or groups or atoms convertible thereto; and

i) V is $(CH_2)_nCR^{13}R^{14}(CH_2)m_1$ $NH_2$ where n is as defined, either $R^{13}$ is $R^4$ as defined or protected hydroxy and $R^{14}$ is H, or $R^{13}$ and $R^{14}$ together are oxo, $m_1$ is m as defined with the proviso that when $R^{13}$ and $R^{14}$ together are oxo $m_1$ is 1; and Y is $ZSO_2$ where Z is a group readily displaceable by a nucleophile;

ii) for compounds of formula (I) where $R^4$ is optionally derivatised hydroxyl, V is $(CH_2)_nCHO$ where n is as defined and Y is $M(CH_2)_mNHSO_2$ where M is a lithium atom or a halomagnesium group and m is as defined; or V is $(CH_2)_n$ M where n and M are as defined and Y is $CHO.(CH_2)_mNHSO_2$ where m is as defined; or

iii) V is H; and Y is $Q(CH_2)_nCR^{13}R^{14}(CH_2)_mNHSO_2$ where Q is a group readily displaceable by a nucleophile and the remaining variables are as hereinbefore defined;

and thereafter as necessary reducing the resulting compound; deprotecting any protected hydroxy or amino group; converting $R^{10}$, $R^{11}$, $R^{12}$ or $R^4$ to $R^1$, $R^2$, $R^3$ or other $R^4$; and optionally salifying or forming an N-oxide the resultant compound of formula (I).

Suitable examples of Z include halide such as chloride or bromide, or hydroxy.

Suitable Q groups are hydroxy, halo such as chloro and bromo; when $R^{13}$ and $R^{14}$ together are oxo and n is O, acyloxy such as $C_{1-4}$ alkanoyloxy, $C_{1-4}$ alkoxycarbonyloxy, and activated hydrocarbyloxy such as pentachlorophenoxy; and when n is 1 to 3, or $R^{13}$ and $R^{14}$ are not together oxo, labile acyloxy such as tosyloxy, mesyloxy or triflate.

It will be appreciated by the skilled man that a protected hydroxyl group is a conventional group readily convertible after a desired reaction to a hydroxyl group, without adversely affecting the rest of the molecule.

Examples of protected hydroxyl include $C_{1-4}$ alkoxy and $C_{1-6}$ alkanoyloxy as defined and described in and under formula (I); benzoyloxy and benzyloxy optionally substituted in the phenyl ring by one or two substituents selected from $C_{1-4}$ alkyl, $C_{1-4}$ alkoxy, trifluoromethyl, halogen and nitro; and tetrahydropyranyloxy. Thus it will be seen that 'derivatised hydroxy', as defined, and 'protected hydroxy' are not coterminous.

Groups $R^{10}$, $R^{11}$ and $R^{12}$ convertible to $R^1$, $R^2$ and $R^3$ will be apparent to the skilled man, but the following are mentioned by way of example:

For $R^{10}$: a hydrogen atom, protected amino such as $C_{1-7}$ carboxylic acylamino, and protected hydroxy such as given hereinbefore for $R^{13}$.

For $R^{11}$ and $R^{12}$: a hydrogen atom, amino and protected hydroxy.

In variant i), the condensation reaction is generally effected at ambient temperature and normal pressure in a solvent inert to both reactants, such as benzene or toluene, optionally in the presence of a base, such as an alkali or alkaline earth metal carbonate, or an appropriate organic base. Use of a base will depend on whether the free-base product or its salt with the acid HZ where Z is as defined is required.

In variant ii), where M is a magnesium (II) halide group, the compound of formula (IX) or (VIII) may be prepared in situ under conventional conditions for Grignard reagents. Those are: reaction of the halide, preferably the bromide, corresponding to the compound of formula (IX) or (VIII) with a molar equivalent or excess of dry, grease-free magnesium particles in a dry ether, for example THF, dimethoxyethane or diethyl ether, free of protic solvents. THF is a preferred solvent. The presence of trace quantities of dibromoethane may be advantageous. Ambient and non-extreme depressed temperatures are suitable, for example between ambient and −15°C, although gentle initiative heating may be advantageous.

When M is lithium, the compound of formula (IX) or (VIII) may be prepared in situ under conventional indirect metallation conditions, for example by reaction of the corresponding halide, preferably the

bromide, with n-butyl lithium. Temperatures of ambient to −60°C are suitable. The completed reaction is conveniently quenched with water.

In variant (iii), if a Q group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at at non-extreme temperature such as −10 to 100°C, for example 0 to 80°C.

If a Q group is a halide or labile acyloxy, then the reaction is preferably carried out at a non-extreme temperature in an inert non-hydroxylic solvent, such as benzene, toluene or diethyl ether. It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate.

If a Q group is acyloxy then the reaction is preferably effected in substantially the same manner as if the leaving group were hydroxy, without a dehydrating catalyst. Suitable examples of acyloxy leaving groups include $C_{1-4}$ alkanoyloxy.

If a Q group is $C_{1-4}$ alkoxycarbonyloxy, then the reaction is preferably carried out in an inert solvent, such as methylene chloride, at a non-extreme temperature in the presence of an acid acceptor such as triethylamine.

If a Q group is activated hydrocarbyloxy, then the reaction is preferably carried out in an inert polar solvent, such as dimethylformamide. It is also preferred that the activated hydrocarbyloxy group is a pentachlorophenyl ester and that the reaction is carried out at ambient temperature.

As regards the deprotection of $R^{13}$ protected hydroxy:

When $R^{13}$ protected hydroxy is $C_{1-4}$ alkoxy deprotection is effectable by conventional methods, such as by boron tribromide or boron triiodide or iodotrimethylsilane. Warm aqueous hydrobromic acid or pyridine hydrochloride may also be used.

When $R^{13}$ is $C_{1-6}$ alkanoyloxy or benzoyloxy optionally substituted as defined deprotection may be effected conventionally, for example by acidic or basic hydrolysis.

When $R^{13}$ is optionally substituted benzyloxy as defined above, or tetrahydropyranyloxy, conversion is effectable by, for example, palladium or platinum-charcoal, at about atmospheric pressure. Non-extreme temperatures at about ambient are generally suitable.

Reduction of the $R^{13}$ and $R^{14}$ oxo function when present to give an $R^4$ hydroxy function may be effected using a strong reductant such as lithium aluminium hydride. This $R^4$ hydroxy group may then be derivatised as discussed hereinafter.

Pharmaceutically acceptable salts, and N-oxides of the compounds of formula (I) may be formed conventionally. The salts may be formed for example by reaction of the base compound of formula (I) with a pharmaceutically acceptable organic or inorganic acid.

N-oxides of the nitrogen atom of the bicyclic ring system are produced by reaction of a compound of formula (I) with an organic peracid, such as m-chloroperbenzoic acid in, for example, a chlorinated hydrocarbon solvent at below ambient temperature.

Quaternary ammonium salts may be prepared by reaction of a compound of formula (I) with the appropriate alkyl, aryl or aralkyl, chloride, bromide or iodide. This reaction may be carried out in a solvent, such as acetone, methanol, ethanol or dimethylformamide, at ambient or elevated temperature with or without pressure.

When L in the compounds of the formula (I) or (VIII) is asymmetrically substituted by one or two methyl groups, the L group has a chiral centre. The point of substitution by $R^4$ is also a chiral centre.

Racemates of compounds of the formula (I), (VIII) or (IX) may be resolved conventionally, e.g., by salification with a chiral acid if appropriate and separation of the resultant salts.

Alternatively, either may be synthesised from corresponding chiral preceeding intermediates.

When L is substituted by two methyl groups, these may be mutually cis or trans about the L ring. A mixture of cis or trans isomers of the compound of formula (I) or (VIII) may be synthesised non stereospecifically and the desired isomer separated conventionally therefrom, e.g. by chromatography; or alternatively the cis or trans isomer may if desired be synthesised from the corresponding cis or trans form of the preceding intermediates.

Compounds of formula (VIII) and (IX) are either known compounds or are novel but preparable analogously to or are routinely derivable from known compounds. Novel compounds of these formulae form an aspect of the present invention.

Cis- and trans- and/or chiral forms of the compounds of the formulae (VIII) or (IX) are either known as separate forms or may be separated conventionally e.g., by chromatography, or resolved conventionally, e.g. by separation of salts with chiral acids.

It will be apparent that the products of the above preparative reaction which contain a group convertible to an $R^1$, $R^2$, $R^3$ or $R^4$ group, whether itself an $R^1$, $R^2$, $R^3$ or $R^4$ group or not, are useful novel intermediates. A number of such conversions is possible not only for the products under discussion, but also for their intermediates as follows:

(a) a hydrogen substituent is convertible to a nitro substituent by nitration;

(b) a nitro substituent is convertible to an amino substituent by reduction;

(c) a $C_{1-7}$ carboxylic acylamino substituent is convertible to an amino substituent by deacylation;

(d) a hydrogen substituent is convertible to a halogen substituent by halogenation;

(e) an amino substituent is convertible to a corresponding substituent substituted by one or two groups selected from $C_{1-4}$ alkyl groups;

(f) a $C_{1-4}$ alkoxy substituent, a phenyl $C_{1-4}$ alkoxy substituent, an in-vivo hydrolysable acyloxy substituent or a protected hydroxyl substituent is convertible to a hydroxyl substituent by deetherification or deesterification as apt;

(g) a hydroxy substituent is convertible to $C_{1-4}$ alkoxy or phenyl $C_{1-4}$ alkoxy by O-alkylation or to in-vivo hydrolysable acyloxy by O-acylation.

Conversions (a) to (g) are only exemplary and not exhaustive of the possibilities.

In regard to (a), nitration is carried out in accordance with known procedures.

In regard to (b), the reduction is carried out with a reagent suitable for reducing nitrobenzene to aniline.

In regard to (c), deacylation is carried out by treatment with a base, such as an alkali metal hydroxide.

In regard to (d), halogenation is carried out with conventional halogenating agents.

In regard to (e), alkylation is carried out with a corresponding alkylating agent such as the chloride or bromide under conventional conditions.

In regard to (f), a $C_{1-4}$ alkoxy substituent is convertible to a hydroxy substituent by conventional methods, such as warming with aqueous hydrobromic acid or by treatment with pyridine hydrochloride, boron tribromide, boron triiodide or iodotrimethylsilane.

A phenyl $C_{1-4}$ alkyl, e.g. benzyl substituent, or a tetrahydropyranyloxy is so convertible by conventional hydrogenolysis.

An in-vivo hydrolysable acyloxy or acyl-protected hydroxy group is so convertible by conventional basic hydrolysis.

In regard to (g), O-alkylation is carried out under conventional conditions in an inert solvent at a non-extreme temperature such as ambient temperature of slightly above or at reflux temperature. The $C_{1-4}$ alkylating agent has a leaving group that is readily displaceable by a nucleophile. Examples of leaving groups include halide, such as chloride, bromide or iodide, or labile acyloxy groups, such as mesyl and tosyl.

O-acylation is carried out under conventional conditions with an acylating agent which has an acyl group capable of forming an in vivo hydrolysable acyloxy group and leaving group, such as a halide, for example chloride and bromide, and hydrogen. When halide is the leaving group, the reaction may be carried out in the presence of a base. When hydroxy is the leaving group, the reaction is generally carried out in the presence of a dehydrating agent, such as dicyclohexylcarbodiimide, in an inert solvent at non-extreme temperature, such as ambient temperature or slightly above, or reflux temperature.

Before carrying out any of these conversions, the effect, if any, on other substituents should be considered, and such reagents as are appropriate should be selected together with the adoption of such precautionary measures as are necessary. For example, O-alkylation and O-acylation may also produce N-alkylated and N-acylated products respectively unless the nitrogen atom(s) is (are) previously protected. This may be conveniently achieved by carrying out the alkylation or acylation reaction in a strong acid, such as trifluoroacetic acid, which protonates, and thereby protects, the nitrogen atom(s).

The following Example illustrates the invention:

## Example

*1-[2-(2,4,5-trichlorobenzenesulphonamido)-propyl]-piperidinium chloride* (2)

Whilst stirring, a solution of 1-(2-amino-propyl)-piperidine (5.7 g; 0.04 mol) and triethylamine (4.1 g; 0.04 mol) in toluene (20 ml) was added dropwise to a solution of 2,4,5-trichlorobenzenesulphonylchloride (11.3 g; 0.04 mol) in toluene (180 ml) at room temperature. Thereafter, the mixture was stirred for a further 6 hours until the reaction was complete. The reaction mixture was extracted twice with water (100 ml). The toluene layer was dried over $Na_2SO_4$ and neutralized with a solution of HCl in ethyl alcohol whereby the reaction product crystallized from the mixture. The precipitate was removed by suction and washed with toluene on the suction filter. The crude chloride was recrystallized from ethanol (300 ml) to obtain the product (13.8 g).

M.p. 233°C

Yield 82% of theoretical

The other compounds of Tables 1 and 2 were prepared analogously.

## Pharmacology of Compounds

Test Procedure to Demonstrate Antiarrythmic Effects

*Electrostimulation Test*

According to the method described by SZEKERES, L. and PAPP G. J., (Naunyn-Schmiedebergs Arch. exp. Path. Pharmak. 245, 70 (1963), arrhythmias are induced in Guinea pigs by electrostimulation of the right ventricle of the heart. The animals are anaesthetized with Urethane (1.2 g/kg i.p.) and artificially respired before a needle electrode is inserted in the right ventrical of the heart. Substances are given intraduodenally 30 min before the stimulation. The voltage needed for induction of extrasystoles in 6

control animals (n=6) is compared with that required for induction of arrhythmias in 6 treated animals (n=6). The difference is statistically evaluated by the unpaired t-test (STUDENT).

This method was used to evaluate the compounds of the present invention. The results are shown in following Tables 1 and 2.

In Tables 1 and 2, * means statistically significant $p<0.05$.

In Tables 1 and 2, the following abbreviations mean recrystallised from the following solvents, or mixtures thereof:

| | |
|---|---|
| DEE | diethyl ether |
| E | ethanol |
| IPA | isopropanol |

No toxic effects were observed in any of the above tests.

TABLE 1

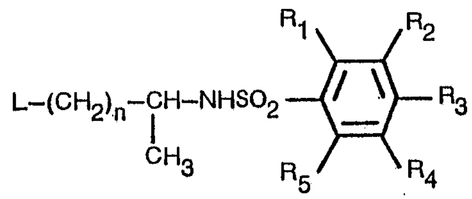

| Compound No. | L | n | $R_1$ | $R_2$ | $R_3$ | $R_4$ | $R_5$ | Salt | Mp°C | Yield | % Increase in Test Voltage |
|---|---|---|---|---|---|---|---|---|---|---|---|
| (1) | 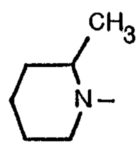 | 3 | $CH_3$ | H | $CH_3$ | H | $CH_3$ | HCl | 147 E/DEE | 38 | 89.4* |
| (2) | 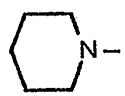 | 1 | Cl | H | Cl | Cl | H | HCl | 233 E | 82 | 29.6* |
| (3) | 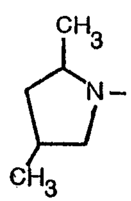 | 3 | $CH_3$ | H | $CH_3$ | H | $CH_3$ | HCl | 173 IPA | 73 | 87.7* |

TABLE 2

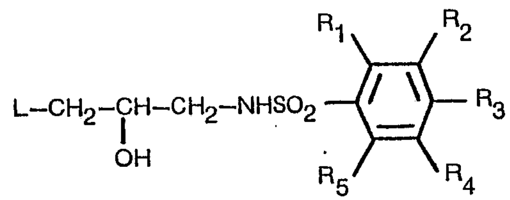

| Compound No. | L | $R_1$ | $R_2$ | $R_3$ | $R_4$ | $R_5$ | Salt | Mp°C | Yield | % Increase in Test Voltage |
|---|---|---|---|---|---|---|---|---|---|---|
| (4) | 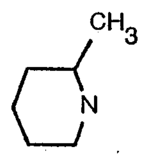 | Cl | H | Cl | Cl | H | HCl | 157 E/DEE | 18 | 60.6* |
| (5) | 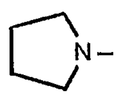 | $CH_3$ | H | $CH_3$ | H | $CH_3$ | HCl | 176 E | 38.5 | 29.5* |
| (6) | 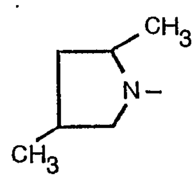 | $CH_3$ | H | $CH_3$ | H | $CH_3$ | HCl | 173 E/DEE | 35.7 | 12.1* |
| (7) | 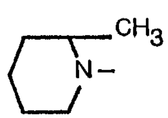 | $CH_3$ | H | $CH_3$ | H | $CH_3$ | HCl | 127 E | 48.6 | 13.4* |

**Claims**

1. A compound of the formula (I) or a pharmaceutically acceptable salt or N-oxide thereof, or a solvate of any of the foregoing:

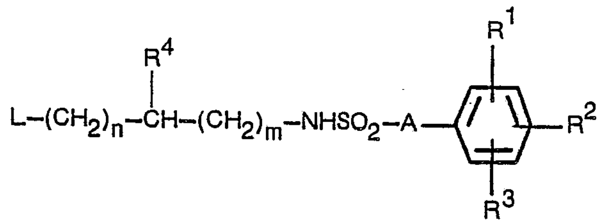

(I)

wherein:

A is a bond, $-CH_2-$ or $-CH=CH-$;

m is 0 or 1;

n is 0 to 3;

$R^4$ is $C_{1-4}$ alkyl, hydroxy, nitrato, $C_{1-4}$ alkoxy, phenyl $C_{1-4}$ alkoxy, or *in vivo* hydrolysable acyloxy;

$R^1$ is halogen, $C_{1-4}$ alkyl, hydroxy, $C_{1-4}$ alkanoyl, nitro, cyano, $CF_3$ or amino optionally substituted by one or two $C_{1-4}$ alkyl groups; and

$R^2$ and $R^3$ are each independently halogen, $C_{1-4}$ alkyl, or hydroxy; and

L is 1-pyrrolidyl, 1-piperidyl, 1-morpholinyl or 4-methyl-1-piperazyl, optionally singly substituted as appropriate by a methyl group at each carbon atom in the 2-, 4- or 6- position up to a total of two methyl substituents in the radical L.

2. A compound according to claim 1 of formula (II):

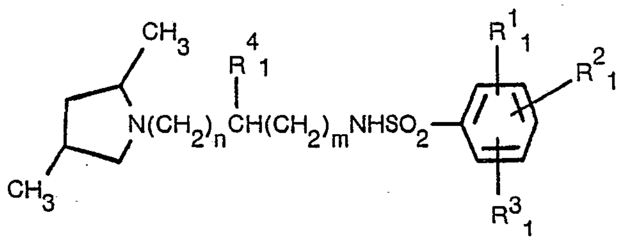

(II)

wherein:

each of $R^1{}_1$, $R^2{}_1$ and $R^3{}_1$ is independently halogen or $C_{1-4}$ alkyl.

$R^4{}_1$ is methyl or hydroxy; and

the remaining variables are as defined in claim 1.

3. A compound according to claim 2 which is
1-[4-(2,4,6-trimethylbenzenesulphonamido)pentyl]-2,4-dimethylpyrrolidine,
1-(3-(2,4,6-trimethylbenzenesulphonamido)-2-hydroxypropyl]-2,4-dimethylpyrrolidine,
or a pharmaceutically acceptable salt or N-oxide thereof, or a solvate of any of the foregoing.

4. A compound according to claim 1 of formula (V):

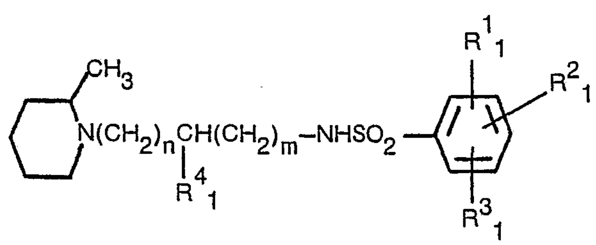

(V)

wherein the variables are as defined in claims 1 and 2.

5. A compound according to claim 4 which is
1-[4-(2,4,6-trimethylbenzenesulphonamido)pentyl]-2-methylpiperidine,
1-[3-(2,4,5-trichlorobenzenesulphonamido)-2-hydroxypropyl]-2-methylpiperidine, or
1-[3-(2,4,6-trimetylbenzenesulphonamido)-2-hydroxypropyl]-2-methylpiperdine,
or a pharmaceutically acceptable salt or N-oxide thereof, or a solvate of any of the foregoing.

6. A compound according to claim 1 which is
1-[2-(2,4,5--trichlorobenzenesulphonamido)propyl]piperidine or
1-[3-(2,4,6-trimethylbenzenesulphonamido)-2-hydroxypropyl]pyrrolidine,
or a pharmaceutically acceptable salt or N-oxide thereof, or a solvate of any of the foregoing.

7. A pharmaceutical composition comprising a compound according to any one of claims 1 to 6 of formula (I), or a pharmaceutically acceptable salt or N-oxide thereof, or a solvate of any of the foregoing, and a pharmaceutically acceptable carrier.

8. A process for the preparation of a compound according to claim 1 of formula (I) or a pharmaceutically acceptable salt or N-oxide thereof, or a solvate of any of the foregoing, which process comprises the reaction of the compounds of formulae (VIII) and (IX):

L—V (VIII)

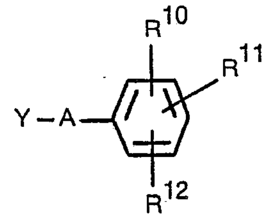

(IX)

wherein

L and A are as defined in claim 1;

$R^{10}$, $R^{11}$ and $R^{12}$ are $R^1$, $R^2$ and $R^{<}$ respectively or groups or atoms convertible thereto; and

i) V is $(CH_2)_nCR^{13}R^{14}(CH_2)_{m1}$ $NH_2$ where n is as defined, either $R^{13}$ is $R^4$ as defined or protected hydroxy and $R^{14}$ is H, or $R^{13}$ and $R^{14}$ together are oxo, $m_1$ is m as defined with the proviso that when $R^{13}$ and $R^{14}$ together are oxo $m_1$ is 1; and Y is $ZSO_2$ where Z is a group readily displaceable by a nucleophile;

ii) for compounds of formula (I) where $R^4$ is optionally derivatised hydroxyl, V is $(CH_2)_nCHO$ where n is as defined and Y is $M(CH_2)_mNHSO_2$ where M is a lithium atom or a halomagnesium group and m is as defined; or V is $(CH_2)_nM$ where n and M are as defined and Y is $CHO.(CH_2)_mNHSO_2$ where m is as defined; or

iii) V is H; and Y is $Q(CH_2)_nCR^{13}R^{14}(CH_2)_mNHSO_2$ where Q is a group readily displaceable by a nucleophile and the remaining variables are as defined;

and thereafter as necessary reducing the resulting compound; deprotecting any protected hydroxy or amino group; converting $R^{10}$, $R^{11}$, $R^{12}$ or $R^4$ to $R^1$, $R^2$, $R^3$ or other $R^4$; and optionally salifying or forming an N-oxide of the resultant compound of formula (I).

9. A compound according to claim 1 of formula (I), or a pharmaceutically acceptable salt thereof, or N-oxide thereof, or a solvate of any of the foregoing, for the treatment or prophylaxis of cardiac arrhythmia.

10. A compound according to claim 1 of formula (I), or a pharmaceutically acceptable salt thereof, or N-oxide thereof, or a solvate of any of the foregoing, for use as an active therapeutic substance.

**Patentansprüche**

1. Eine Verbindung der Formel (I)

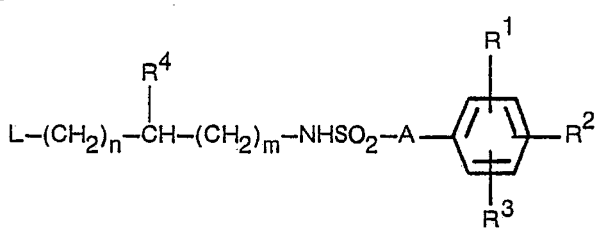

(I)

oder ein pharmazeutisch zulässiges Salz oder ein N-Oxid derselben oder ein Solvat irgendeiner der vorstehend genannten Verbindungen

wobei

A eine Bildung ist oder eine Gruppe $—CH_2—$ oder eine Gruppe —CH=CH—;

m den Wert 0 oder 1 hat;

n einen Wert von 0 bis 3 hat;

$R^4$ $C_{1-4}$-Alkyl, Hydroxy, Nitrato, $C_{1-4}$-Alkoxy, Phenyl-$C_{1-4}$-alkoxy oder in vivo hydrolysierbares Acyloxy ist;

$R^1$ Halogen, $C_{1-4}$-Alkyl, Hydroxy, $C_{1-4}$-Alkanoyl, Nitro, Cyano, $CF_3$ oder gegebenenfalls mit ein oder zwei $C_{1-4}$-Alkylgruppen substituiertes Amino bedeutet;

$R^2$ und $R^3$ jeweils unabhängig voneinander Halogen, $C_{1-4}$-Alkyl oder Hydroxy darstellen und

L 1-Pyrrolidyl, 1-Piperidyl, 1-Morpholinyl oder 4-Methyl-1-piperazyl ist, wobei diese Gruppen gegebenenfalls einzeln, wo angemessen, an jedem Kohlenstoffatom in 2-, 4-, oder 6-Stellung durch eine Methylgruppe substituiert sein können und zwar bis zu einer Gesamtheit von zwei Methylsubstituenten in dem Radikal L.

2. Eine Verbindung gemäß Anspruch 1 der Formel (II),

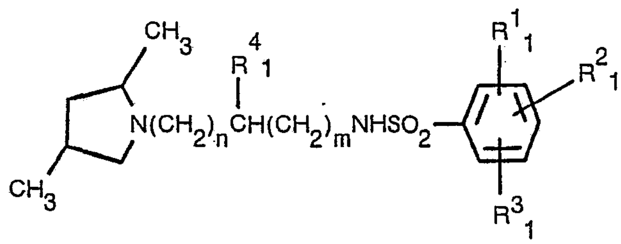

(II)

in welcher

$R^1_1$, $R^2_1$ und $R^3_1$ unabhängig voneinander Halogen oder $C_{1-4}$Alkyl bedeuten,

$R^4_1$ Methyl oder Hydroxy ist und die verbleibenden Variablen wie in Anspruch 1 definiert sind.

3. Eine Verbindung gemäß Anspruch 2, nämlich

1-[4-(2,4,6-Trimethylbenzolsulphonamido)pentyl]-2,4-dimethylpyrrolidin bzw.

1-[3-(2,4,6-Trimethylbenzolsulphonamido)-2-hydroxypropyl]-2,4-dimethylpyrrolidin

oder ein pharmazeutisch zulässiges Salz oder N-Oxid dieser Verbindungen oder ein Solvat irgendeiner der vorstehenden Verbindungen.

4. Eine Verbindung gemäß Anspruch 1 der Formel (V),

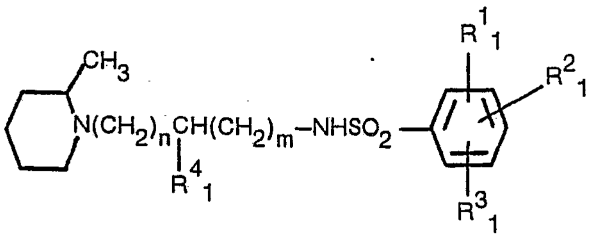

(V)

in welcher die Variablen wie in den Ansprüchen 1 und 2 definiert sind.

5. Eine Verbindung gemäß Anspruch 4, nämlich

1-[4-(2,4,6-Trimethylbenzolsulphonamido)pentyl]-2-methylpiperidin,

1-[3-(2,4,5-Trichlorbenzolsulpohonamido)-2-hydroxypropyl]-2-methylpiperidin, oder

1-[3-(2,4,6-Trimethylbenzolsulphonamido)-2-hydroxypropyl]-2-methylpiperidin,

oder ein pharmazeutisch zulässiges Salz oder N-Oxid dieser Verbindungen oder ein Solvat der vorstehend genannten Verbindungen.

6. Eine Verbindung gemäß Anspruch 1, nämlich

1-[2-(2,4,5-Trichlorbenzolsulphonamido)propyl piperidin oder

1-[3-(2,4,6-Trimethylbenzolsulphonamido)-2-hydroxypropyl]pyrrolidin,

oder ein pharmazeutisch zulässiges Salz oder N-Oxid dieser Verbindungen oder ein Solvat irgendeiner der vorstehend genannten Verbindungen.

7. Eine pharmazeutisch Zusammensetzung enthaltend eine Verbindung gemäß irgendeinem der Ansprüche 1 bis 6 mit der Formel (I) oder ein pharmazeutisch zulässiges Salz oder N-Oxid solcher Verbindungen oder ein Solvat irgendeiner der vorstehend genannten Verbindungen und einen pharmazeutisch zulässigen Träger.

8. Ein Verfahren zur Herstellung einer Verbindung gemäß Anspruch 1 mit Formel (I), oder eines pharmazeutisch zulässigen Salzes oder N-Oxids derselben, oder eines Solvats irgendeiner der vorstehend genannten Verbindungen, welches Verfahren die Umsetzung von Verbindungen, welches Verfahren die Umsetzung von Verbindungen der Formeln (VIII) und (IX) umfaßt,

L–V (VIII)

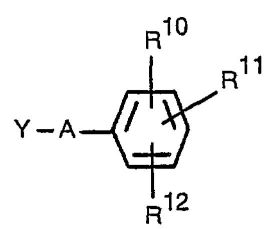

(IX)

in welchen

L und A wie in Anspruch 1 definiert sind;

$R^{10}$, $R^{11}$ und $R^{12}$ identisch sind mit $R^1$, $R^2$ und $R^3$ bzw. in diese Gruppen umwandelbare Gruppen oder Atome bedeuten und

i) V die Bedeutung $(CH_2)_nCR^{13}R^{14}(CH_2)_{m1}$ $NH_2$ hat, wobei n wie vorstehend definiert ist, entweder $R^{13}$ der Gruppe $R^4$, wie vorstehend definiert, entspricht oder eine mit einer Schutzgruppe versehene Hydroxy-Gruppe bedeutet und $R^{14}$ Wasserstoff ist oder $R^{13}$ und $R^{14}$ zusammen eine Oxo-Gruppe darstellen, $m_1$ die Bedeutung von m wie vorstehend definiert hat, mit der Maßgabe, daß wenn $R^{13}$ und $R^{14}$ zusammen die

Oxo-Gruppe bedeuten, $m_1$ den Wert 1 hat, und Y die Bedeutung $ZSO_2$ hat, wobei Z eine durch eine Nukleophil leicht verdrängbare Gruppe bedeutet;

ii) für Verbindungen der Formel (I), in denen $R^4$ Hydroxyl oder Hydroxyderivate bedeutet, V die Gruppe $(CH_2)_nCHO$ ist, wobei n wie vorstehend definiert ist und Y die Gruppe $M(CH_2)_mNHSO_2$ bedeutet, wobei M ein Lithiumatom oder eine Halogenmagnesium-Gruppe ist und m wie vorstehend definiert ist oder V die Gruppe $(CH_2)_nM$, wobei n und M wie vorstehend definiert sind und Y die Gruppe $CHO.(CH_2)_mNHSO_2$ bedeutet, wobei m wie vorstehend definiert ist oder

iii) V Wasserstoff ist und Y die nachstehende Gruppe bedeutet $Q(CH_2)_nCR^{13}R^{14}(CH_2)_mNHSO_2$, wobei Q eine durch ein Nukleophil leicht verdrängbare Gruppe ist und die anderen Variablen wie vorstehend definiert sind;

und umfassend, falls erforderlich, die Reduktion der entstehenden Verbindung, die Abspaltung von Schutzgruppen aus gegebenenfalls vorliegenden, mit Schutzgruppen versehenen Hydroxy- oder Amino-Gruppen, das Umwandeln der Gruppen $R^{10}$, $R^{11}$, $R^{12}$ oder $R^4$ in entsprechende Gruppen $R^1$, $R^2$, $R^3$ oder anderes $R^4$ und gegebenenfalls Salzbildung oder die Bildung eines N-Oxids der so gebildeten Verbindung der Formel (I).

9. Verwendung einer Verbindung gemäß Anspruch 1 mit der Formel (I) oder eines pharmazeutisch zulässigen Salzes oder eines N-Oxids derselben oder eines Solvates irgendeiner der vorstehend genannten Verbindungen für die Behandlung oder Prophylaxe von Herzrythmusstörungen.

10. Verwendung einer Verbindung gemäß Anspruch 1 mit der Formel (I) oder eines pharmazeutisch zulässigen Salzes oder N-Oxids oder Solvates irgendeiner der vorstehend genannten Verbindungen als therapeutisch aktiver Wirkstoff.

**Revendications**

1. Composé de formule (I), ou un sel ou un N-oxyde de celui-ci, acceptable du point de vue pharmaceutique, ou un solvate d'un quelconque des composés précédents

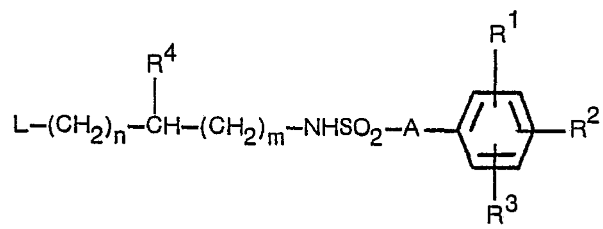

(I)

dans laquelle

A est une liaison, un groupe $—CH_2—$ ou $—CH{=}CH—$;

m est 0 ou 1;

n est 0 à 3;

$R^4$ est un groupe alcoyle en $C_{1-4}$, hydroxy, nitrato, alcoxy en $C_{1-4}$, phényl-alcoxy en $C_{1-4}$ ou acyloxy hydrolysable in vivo;

$R^1$ est un atome d'halogène, un groupe alcoyle en $C_{1-4}$, hydroxy, alcanoyle en $C_{1-4}$, nitro, cyano, $CF_3$ ou amino éventuellement substitué par un ou deux groupes alcoyles en $C_{1-4}$; et

$R^2$ et $R^3$ sont chacun indépendamment un atome d'halogène, un groupe alcoyle en $C_{1-4}$ ou hydroxy; et

L est un groupe 1-pyrrolidyle, 1-pipéridyle, 1-morpholinyle ou 4-méthyl-1-pipérazyle, dont chaque atome de carbone en position 2-, 4- ou 6- est éventuellement substitué de façon appropriée par un seul groupe méthyle, avec un total maximum de 2 substituants méthyles dans le radical L.

2. Composé suivant la revendication 1, représenté par la formule (II)

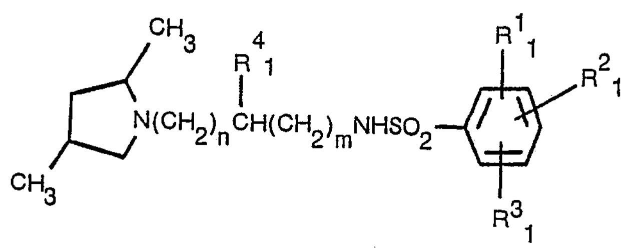

(II)

dans laquelle:

chacun des $R^1_1$, $R^2_1$ et $R^3_1$ est indépendamment un atome d'halogène ou un groupe alcoyle en $C_{1-4}$;

$R^4_1$ est un groupe méthyle ou hydroxy; et

les autres variables sont telles qu'elles sont définies dans la revendication 1.

3. Composé suivant la revendication 2, qui est la 1-[4-(2,4,6-triméthylbenzènesulfonamido)pentyl]-2,4-diméthylpyrrolidine, la 1-[3-(2,4,6-triméthylbenzènesulfonamido)-2-hydroxypropyl]-2,4-diméthylpyrrolidine ou

un sel, ou un N-oxyde de celles-ci acceptables du point de vue pharmaceutique ou un solvate de l'un quelconque de ces composés.

4. Composé suivant la revendication 1, représentée par la formule (V)

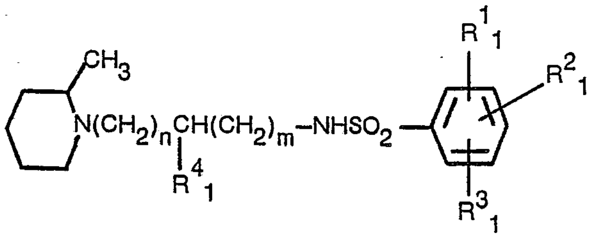

(V)

dans laquelle les variables sont telles qu'elles sont définies dans les revendications 1 et 2.

5. Composé suivant la revendication 4, qui est
la 1-[4-(2,4,6-triméthylbenzènesulfonamido)pentyl]-2-méthylpipéridine,
la 1-[3-(2,4,5-trichlorobenzènesulfonamido)-2-hydroxypropyl]-2-méthylpipéridine, ou
la 1-[3-(2,4,6-triméthylbenzènesulfonamido)-2-hydroxypropyl]-2-méthylpipéridine,
ou un sel ou un N-oxyde acceptable du point de vue pharmaceutique de celles-ci, ou un solvate de l'un quelconque de ces composés.

6. Composé suivant la revendication 1, qui est
la 1-[2-(2,4,5-trichlorobenzènesulfonamido)propyl]pipéridine, ou
la 1-[3-(2,4,6-triméthylbenzènesulfonamido)-2-hydroxypropyl]pyrrolidine,
ou un sel ou un N-oxyde de celles-ci acceptables du point de vue pharmaceutique ou un solvate de l'un quelconque de ces composés.

7. Composition pharmaceutique comprenant un composé suivant l'une quelconque des revendications 1 à 6, représenté par la formule (I), ou un sel ou un N-oxyde de celui-ci acceptable du point de vue pharmaceutique ou un solvate de l'un quelconque de ces composés, et un support acceptable du point de vue pharmaceutique.

8. Procédé de préparation d'un composé suivant la revendication 1 représenté par la formule (I), ou d'un sel ou d'un N-oxyde de celui-ci acceptable du point de vue pharmaceutique ou d'un solvate de l'un quelconque des composés ci-dessus, caractérisé en ce qu'il comprend la réaction des composés de formules (VIII) et (IX)

L—V (VIII)

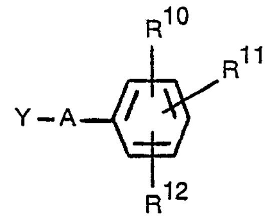

(IX)

dans lesquelles

L et A sont tels que définis dans la revendication 1,

$R^{10}$, $R^{11}$ et $R^{12}$ sont $R^1$, $R^2$ et $R^3$ respectivement ou des groupes ou des atomes pouvant être convertis en ceux-ci; et

(i) V est le radical $(CH_2)_nCR^{13}R^{14}(CH_2)m_1$ $NH_2$ où n est tel que défini, et, soit $R^{13}$ est $R^4$ tel que défini ou un groupe hydroxy protégé et $R^{14}$ est un atome d'hydrogène soit $R^{13}$ et $R^{14}$ forment ensemble un groupe oxo, $m_1$ est m tel que défini, à condition que dans le cas où $R^{13}$ et $R^{14}$ forment ensemble un groupe oxo, $m_1$ est 1; et Y est $ZSO_2$ où Z est un groupe facilement déplacé par un nucléophile;

(ii) pour des composés de formule (I) dans laquelle $R^4$ est un groupe hydroxy éventuellement un dérivé de celui-ci, V est un radical $(CH_2)$ CHO, où n est tel que défini et Y est $M(CH_2)_mNHSO_2$ où M est un atome de lithium ou un groupe halomagnésien et m est tel que défini; ou V est $(CH_2)_nM$, où n et M sont tels que définis et Y est un groupe $CHO.(CH_2)_mNHSO_2$ où n est tel que défini; ou

(iii) V est un atome d'hydrogène; et Y est un radical $Q(CH_2)_nCR^{13}R^{14}(CH_2)_mNHSO_2$ où Q est un groupe facilement déplacé par un nucléophile et les autres variables sont telles que définies;

et ensuite, si cela est nécessaire, la réduction du composé résultant, la déprotection d'un quelconque groupe amino ou hydroxy protégé; la conversion de $R^{10}$, $R^{11}$, $R^{12}$ ou $R^4$ en $R^1$, $R^2$, $R^3$ ou autre $R^4$; et éventuellement la formation d'un sel ou d'un N-oxyde du composé résultant de formule (I).

9. Composé suivant la revendication 1, représenté par la formule (I) ou un sel ou un N-oxyde de celui-ci acceptable du point de vue pharmaceutique, ou un solvate de l'un quelconque des composés ci-dessus, pour le traitement ou la prophylaxie de l'arythmie cardiaque.

10. Composé suivant la revendication 1, représenté par la formule (I) ou un sel ou un N-oxyde de celui-ci acceptable du point de vue pharmaceutique, ou un solvate de l'un quelconque de ces composés, utile comme substance thérapeutique active.